# FASCICULE DE BREVET EUROPEEN

(11) **EP 1 965 786 B1**
(45) Date de publication et mention de la délivrance du brevet: **11.11.2015**
(21) Numéro de dépôt: 06842050.4
(22) Date de dépôt: 28.11.2006
(51) Int. Cl.: A61K 31/19, A61P 17/00

(54) **COMPOSITIONS COMPRENANT AU MOINS UN COMPOSE RETINOIDE ET AU MOINS UN COMPOSE ANTI-IRRITANT ET LEURS UTILISATIONS**
ZUSAMMENSETZUNGEN MIT MINDESTENS EINER RETINOID-VERBINDUNG UND MINDESTENS EINER REIZHEMMENDEN VERBINDUNG SOWIE ANWENDUNGEN DAVON
COMPOSITIONS INCLUDING AT LEAST ONE RETINOID COMPOUND AND AT LEAST ONE ANTI-IRRITANT COMPOUND AND USES THEREOF

(30) Priorité: 15.12.2005 FR 0512761
(43) Date de publication de la demande: 10.09.2008
(73) Titulaire: GALDERMA RESEARCH & DEVELOPMENT, 06410 Biot (FR)
(72) Inventeur: FREDON, Laurent, F-06330 Roquefort Les Pins (FR); MALLARD, Claire, F-06250 Mougins (FR); FERRARA, Eve, F-06560 Valbonne (FR)
(74) Mandataire: Starck-Loudes, Anne-Caroline
(86) Numéro de dépôt international: PCT/FR2006/051239
(87) Numéro de publication internationale: WO 2007/071860

(56) Documents cités:
- ES-A1- 2 137 125
- GB-A- 950 777
- FROM CIRD GALDERMA ET AL: "Pharmacology and chemistry of adapalene" JOURNAL OF THE AMERICAN ACADEMY OF DERMATOLOGY, C.V. MOSBY, ST. LOUIS, MO, US, vol. 36, no. 6, juin 1997 (1997-06), pages S96-S103, XP005177982 ISSN: 0190-9622
- EGGENSPERGER H ET AL: "Glycyrrhetinsäure - und was man von einem multiaktiven Wirkstoff für die Kosmetik und Dermatologie erwarten darf" SOFW-JOURNAL SEIFEN, OELE, FETTE, WACHSE, VERLAG FUR CHEMISCHE INDUSTRIE, AUGSBURG, DE, vol. 122, no. 14, 1996, pages 990-997, XP002257130 ISSN: 0942-7694
- PATENT ABSTRACTS OF JAPAN vol. 2003, no. 12, 5 décembre 2003 (2003-12-05) & JP 2005 179318 A (POLA CHEM IND INC), 7 juillet 2005 (2005-07-07)
- DATABASE WPI DERWENT PUBLICATIONS LTD., LONDON, GB; cn1359904 UNIV NINGXIA: "Preparation of potassium glycyrrhetate, useful for reducing blood fat, or treating inflammation , hypersensitivity, virus, cough or asthma, comprises hydrolysis of glcyrrhizic acid and reaction with potassium hydroxide." XP002399708 Database accession no. 2002-658830

## Description

La présente invention concerne des compositions pour application topique, et leurs utilisations en tant que produits cosmétiques ou pharmaceutiques, lesdites compositions étant destinées, en particulier, au traitement de l'acné.

L'acné est une pathologie multifactorielle fréquente qui atteint la peau riche en glandes sébacées (visage, région scapulaire, bras et régions intertrigineuses). Elle est la plus fréquente des dermatoses. Les cinq facteurs pathogéniques suivants jouent un rôle déterminant dans la constitution de l'acné :
1. la prédisposition génétique;
2. la surproduction de sébum (séborrhée);
3. les androgènes;
4. les troubles de la kératinisation folliculaire (comédogénèse); et
5. la colonisation bactérienne et les facteurs inflammatoires.

Il existe plusieurs formes d'acnés, ayant toutes en commun l'atteinte des follicules pilosébacés. On peut citer notamment, l'acné conglobata, l'acné chéloïde de la nuque, l'acné médicamenteuse, l'acné miliaire récidivante, l'acné nécrotique, l'acné neonatorum, l'acné prémenstruelle, l'acné professionnelle, l'acné rosacée, l'acné sénile, l'acné solaire, et l'acné vulgaire.

L'acné vulgaire, appelée également acné juvénile polymorphe, est la plus courante. Elle comprend quatre stades :
- Le stade 1 correspond à l'acné comédonienne caractérisée par un grand nombre de comédons ouverts et/ou fermés, et de microkystes.
- Le stade 2, ou acné papulopustuleuse, est de gravité légère à modérée. Elle est caractérisée par la présence de comédons ouverts et/ou fermés, de microkystes, mais également de papules rouges et de pustules. Elle touche principalement le visage et laisse peu de cicatrices.

- Le stade 3, ou acné papulocomédonienne, est plus grave et s'étend au dos, au thorax et aux épaules. Elle est accompagnée d'un plus grand nombre de cicatrices.
- Le stade 4, ou acné nodulokystique, s'accompagne de nombreuses cicatrices.
Elle présente des nodules ainsi que des pustules volumineuses violacées et douloureuses.

Les différentes formes d'acné décrites précédemment peuvent être traitées par des actifs tels que les anti-séborrhéiques et les anti-infectieux, par exemple le peroxyde de benzoyle (notamment le produit Eclaran® commercialisé par la société Pierre Fabre), par des rétinoïdes tels que la trétinoïne (notamment le produit Retacnyl® commercialisé par la société Galderma) ou l'isotrétinoïne (produit Roaccutane® commercialisé par les Laboratoires Roche), ou encore par des dérivés d'acide naphtoïque. Les dérivés d'acide naphtoïque, tels que notamment l'acide 6-[3-(1-adamantyl)-4-méthoxyphényl]-2-naphtoïque), communément appelé adapalène (produit Differine® commercialisé par la société Galderma), sont largement décrits et reconnus comme des principes actifs aussi efficaces que la trétinoïne pour le traitement de l'acné.

L'adapalène en particulier présente une efficacité unanimement avérée ; cependant, il serait avantageux et utile que sa tolérance par voie topique, bien que supérieure à celle de ses concurrents appartenant à la même classe chimique (trétinoïne, tazarotène), soit améliorée.

Le brevet ES 2 137 125 divulgue l'utilisation de sel de zinc de l'acide 18p-glycyrrhétinique pour son effet anti-inflammatoire et anti-acné. Toutefois, ce brevet ne divulgue pas d'association du sel avec l'adapalène.

Or, la Demanderesse a découvert de façon surprenante que l'association d'adapalène avec le sel de potassium de l'acide 18p-glycyrrhétinique peut améliorer significativement la tolérance de ce rétinoïde, et ainsi pallier au problème d'irritation. En effet, comme le montre l'exemple 2, le sel de potassium de l'acide 18p-glycyrrhétinique permet de réduire l'oedème provoqué par l'adapalène jusqu'à plus de 60%.

La présente invention a donc pour objet une composition, notamment pharmaceutique, et de préférence dermatologique, destinée notamment à une application topique, comprenant, dans un milieu physiologiquement acceptable, au moins un composé rétinoïde qui est l'adapalène, et au moins un composé anti-irritant qui est le sel de potassium de l'acide 18p-glycyrrhétinique.

Avantageusement, la composition ne comprend aucun agent dépigmentant distinct de l'adapalène.

Par milieu physiologiquement acceptable, on entend un milieu compatible avec la peau, les muqueuses et/ou les phanères.

Dans les compositions selon l'invention, la concentration en composé rétinoïde (adapalène) est comprise entre 0,001% et 10%, préférentiellement entre 0,01% et 5% et, plus préférentiellement, entre 0,05% et 2% en poids du poids total de la composition. Dans l'ensemble du présent texte, à moins qu'il ne soit spécifié autrement, il est entendu que lorsque des intervalles de concentrations sont donnés, ils incluent les bornes supérieure et inférieure dudit intervalle.

De préférence, la concentration en composé rétinoïde (adapalène) est égale à 0,1%. De façon alternative, la concentration en composé rétinoïde est de préférence égale à 0,3%.

La concentration en composé anti-irritant (sel de potassium de l'acide 18p-glycyrrhétinique) est quant à elle comprise entre 0,01% et 10%, préférentiellement entre 0,1% et 7%.

Les compositions selon la présente invention peuvent se présenter sous toutes les formes galéniques normalement utilisées pour une application topique, notamment sous forme de dispersions aqueuses, hydroalcooliques ou huileuses, de dispersions du type lotion, de gels aqueux, anhydres ou lipophiles, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-liquide ou solide du type crème, gel-crème, mousse ou pommade ou de micro émulsions, de micro capsules, de micro particules ou de dispersions vésiculaires de type ionique et/ou non ionique, ou encore sous forme de sprays.

De préférence, les compositions se présentent sous la forme d'un gel. L'homme du métier veillera à choisir les excipients constituant les compositions selon l'invention en fonction de la forme galénique souhaitée et de manière à ce que les propriétés avantageuses de la composition selon l'invention soient respectées.

La composition selon l'invention peut en outre notamment comprendre un ou plusieurs des ingrédients suivants :
a) un ou plusieurs agents gélifiants ou agents de suspension,
b) un ou plusieurs agents chélatants,
c) un ou plusieurs agents mouillants,
d) un ou plusieurs agents conservateurs.

A titre d'exemple non limitatif de gélifiants ou agent de suspension pouvant entrer dans les compositions selon l'invention, on peut citer les carbomers vendus sous le nom générique de Carbopol®, les carbomers dits non sensibles aux électrolytes, vendus sous le nom d'Ultrez 10® ou de Carbopol ETD® par la société BF Goodrich, les polysaccharides avec à titre d'exemples non limitatifs la gomme de xanthane telle que le Keltrol T® vendu par la société Kelco, la gomme guar, les chitosans, la cellulose et ses dérivés tel que l'hydroxyéthylcellulose, en particulier, le produit vendu sous le nom de Natrosol HHX 250® par la société Aqualon, et le copolymère d'acrylamide de sodium et d'acrylamino-2-méthylpropane sulphonate en dispersion à 40% dans l'isohexadécane et le polysorbate 80 vendu sous le nom de Simulgel 600® par la société Seppic.

A titre de gélifiant préféré, on peut citer l'hydroxyéthylcellulose vendue notamment sous le nom Natrosol HHX 250®.

Parmi les agents chélatants, on peut citer à titre d'exemples non limitatifs l'acide diéthylène triamine pentaacétique (DTPA), l'acide éthylène diamine-di (O-hydroxyphényl acétique) (EDDHA), l'acide hydroxy-2-éthylène diamine triacétique (HEDTA), l'acide éthyldiamine-di (O-hydroxy-p-méthyl phényl) acétique (EDDHMA), l'acide éthylène diamine tetraacétique (EDTA) et l'acide éthylène diamine-di (5-carboxy-2-hydroxyphényl) acétique (EDDCHA).

Parmi les agents mouillants qui ont pour rôle de diminuer la tension superficielle et de permettre un plus grand étalement du liquide, on utilise préférentiellement, sans que cette liste soit limitative, des composés tels que le propylène glycol, le dipropylène glycol, le propylène glycol dipélargonate, le lauroglycol et l'éthoxydiglycol, seuls ou en mélange. On peut également citer des composés, connus par ailleurs pour leur rôle d'émulsifiants, tels que le Tween 80, Glyceryl Monostearate & POE Stearate vendu sous le nom Arlacel 165FL® par la société Uniquema, Polyoxyethylene (21) Stearyl Ether vendu sous le nom Brij721® par la société Uniquema, les synperonics avec notamment le Synperonic PE/L62 (Poloxamer 182) ou le Synperonic PE/L44 (Poloxamer 124).

A titre d'agent mouillant préféré, on peut citer le propylène glycol, le Synperonic PE/L62 (Poloxamer 182) ou le Synperonic PE/L44 (Poloxamer 124).

Parmi les agents conservateurs, on peut citer à titre d'exemples non limitatifs l'acide benzoïque et ses dérivés avec l'alcool benzylique, le chlorure de benzalkonium, le benzoate de sodium, le bronopol, la chlorhexidine, le chlorocrésol et ses dérivés, l'alcool éthylique, l'alcool phénéthylique, le phénoxyéthanol, le sorbate de potassium, la diazolidinylurée, les parabènes tels que le propyl parabène ou le méthyl parabène, pris seuls ou en mélanges.

A titre d'agent conservateur préféré, on peut citer les parabènes et le phénoxyéthanol ou le chlorure de benzalkonium seuls ou en mélange.

La composition selon l'invention peut comprendre également un ou plusieurs émulsionnants.

Les émulsionnants tensio-actifs sont des composés amphiphiles qui possèdent une partie hydrophobe ayant une affinité pour l'huile et une partie hydrophile ayant une affinité pour l'eau créant ainsi un lien entre les deux phases. Les émulsionnants ioniques ou non ioniques stabilisent donc les émulsions huile/eau en s'adsorbant à l'interface et en formant des couches lamellaires de cristaux liquides.

A titre d'émulsifiants préférés on peut citer les émulsionnants cités préalablement pour leur propriété d'agents mouillants ou des émulsifiants lipophiles de type Glucate SS et Glucamate SSE..

Les compositions de l'invention peuvent comprendre en outre tout additif usuellement utilisé dans le domaine cosmétique ou pharmaceutique tel que des neutralisants, des filtres solaires, des antioxydants, des charges, des électrolytes, des colorants, des bases ou acides usuels, minéraux ou organiques, des parfums, des huiles essentielles, des actifs cosmétiques, des hydratants, des vitamines, des acides gras essentiels, des sphingolipides, des composés autobronzants tels que la DHA, des agents apaisants et protecteurs de la peau, des agents propénétrants, ou un mélange de ceux-ci. Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées.

Ces additifs peuvent être présents dans la composition à raison de 0,001% à 20 % en poids par rapport au poids total de la composition.

La présente invention a également pour objet la composition telle que décrite précédemment à titre de médicament.

En particulier, l'invention se rapporte à l'utilisation d'une composition telle que décrite précédemment pour la préparation d'un médicament destiné au traitement et/ou à la prévention des affections dermatologiques liées à un désordre de la kératinisation portant sur la différenciation et sur la prolifération cellulaire, notamment pour traiter les acnés vulgaires, comédoniennes, papulopustuleuse, papulocomédoniennes, nodulokystiques, les acnés conglobata, les acnés chéloïdes de la nuque, les acnés miliaires récidivantes, les acnés nécrotiques, les acnés neonatorum, les acnés professionnelles, les acnés rosacées, les acnés séniles, les acnés solaires et les acnés médicamenteuses.

De préférence, l'invention se rapporte à l'utilisation d'une composition telle que décrite précédemment pour la préparation d'un médicament destiné à prévenir et/ou à traiter les acnés vulgaires.

Préférentiellement, lesdites compositions selon l'invention sont administrées par voie topique.

En outre, l'invention porte également sur l'utilisation cosmétique non thérapeutique d'une composition selon l'invention pour lutter contre l'aspect gras de la peau ou des cheveux.

### Exemple 1 : Solutions d'anti-irritants

Les anti-irritants utilisés sont formulés sauf indication contraire dans un véhicule éthanol/eau (50 :50) aux concentrations indiquées dans le tableau ci-dessous. Ce dernier indique également, pour chaque anti-irritant, le groupe traité dans l'exemple 2.

| **Groupe** | **Anti-irritants** | **Teneur en solution hydroalcoolique 50 :50 (%)** |
|---|---|---|
| 4 | acide 18β-glycyrrhétinique (Enoxolone) | 1.5 |
| 5 | sel de potassium de l'acide 18β-glycyrrhétinique | 1.5 |

Le groupe 4 est utilisé comme témoin négatif dans les études qui suivent. En effet, comme le montrent les études suivantes, bien qu'étant connu comme anti-irritant, l'enoxolone n'a pas d'effet sur l'irritation due aux rétinoïdes.

### Exemple 2 : Evaluation de l'effet de différents anti-irritants en traitement préventif avant application topique de Différine® gel chez la souris BALB/c : étude de tolérance

La présente étude a pour but de comparer le pouvoir irritant d'un gel de référence à 0,1% d'adapalène lorsque ce traitement est précédé ou non d'un traitement par un anti-irritant.

Le traitement consiste en une application topique quotidienne (20 µl) d'anti-irritant formulé dans un véhicule hydro-alcoolique (50% d'éthanol et 50% d'eau en volume) sur la face interne de l'oreille droite de souris BALB/c réparties en cinq groupes (souris femelles âgées de 9 semaines environ), suivie par une application topique (20 µl) de Différine® gel (gel de référence à 0,1% d'adapalène), à raison d'une application de chaque formulation par jour pendant 6 jours.

Les produits à tester sont :
Groupe 1 : Non traités (témoins)
Groupe 2 : Différine® gel (gel de référence)
Groupe 3 : Solution Ethanol/Eau (véhicule hydro-alcoolique des anti-irritants) puis Différine® gel
Groupe 4 : Enoxolone puis Différine® gel
Groupe 5 : Sel de potassium de l'acide 18β-glycyrrhétinique puis Différine® gel

L'évaluation se fait par des mesures de l'épaisseur de l'oreille à l'aide de l'Oditest et par observation clinique des animaux du 2ème au 19ème jour.

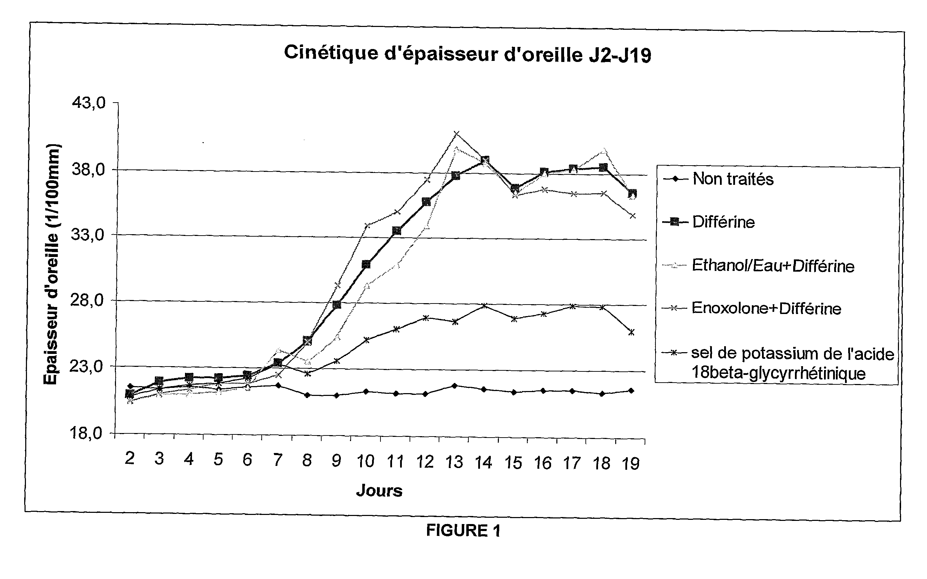

Les résultats sont représentés dans le tableau ci-dessous et dans la figure 1 :
- La figure 1 représente les cinétiques de l'épaisseur moyenne des oreilles de souris entre le 2ème et 19ème jours pour les groupes 1 à 3 (références) et 4 et 5.

Ces cinétiques montrent que la formulation Différine® gel seule est irritante ; l'ajout du véhicule hydro-alcoolique à cette formulation ne change pas le degré d'irritation (groupe 3).
L'enoxolone (groupe 4) n'a quasiment aucun effet sur la diminution de l'irritation provoquée par Différine® gel.
En revanche, l'application de sel de potassium de l'acide 18β-glycyrrhétinique (groupe 5) diminue significativement l'irritation provoquée par Différine® gel ; cette diminution est supérieure à 60%.

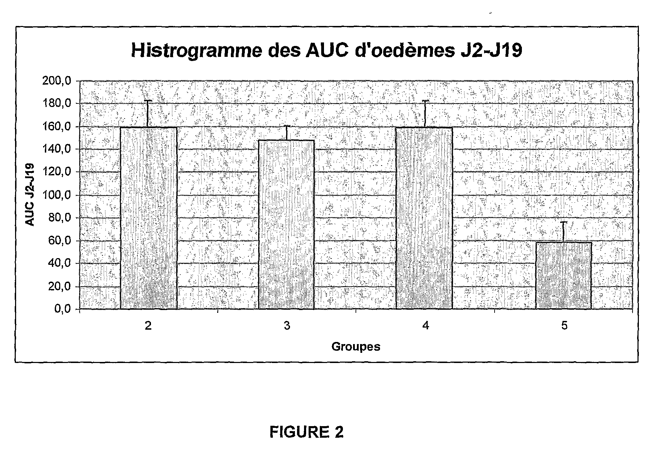

**Tableau récapitulatif des résultats des aires sous la courbe (AUC) des cinétiques des épaisseurs d'oreille (cf Figure 2 pour les histogrammes)**

| | AUC d'oedème D2-D19 | | % inhibition AUC vs Differin | P valeurs | t-test Student vs Differin |
|---|---|---|---|---|---|
| | Moyenne | sem | | | |
| Non traités | | | | | |
| Différine | 158,6 | 24,4 | | | |
| Ethanol / eau + Différine | 147,6 | 12,8 | **7,0** | **0,6989** | **NS** |
| enoxolone + Différine | 159,0 | 23,8 | **-0,3** | **0,9909** | **NS** |
| Sel de potassium de l' acide 18β-glycyrrhétinique + Différine | 58,2 | 17,5 | **63,3** | **0,0102** | ***** |

| | | | | | |
|---|---|---|---|---|---|
| **NS=Non Significatif** | | | | | |

Les résultats de l'étude montrent qu'après applications topiques répétées de 20µl d'une solution d'anti-irritant puis de 20µl Différine® gel de J1 à J6 sur l'oreille des souris BALB/c :
- La formulation Différine® gel est irritante ;
- L'enoxolone n'a pas d'effet sur l'irritation provoquée par Différine® gel ;
- En revanche, l'anti-irritant sel de potassium de l'acide 18β-glycyrrhétinique diminue de 63% l'oedème.

Cet exemple démontre que des anti-irritants potentiels n'ont pas tous le même effet vis-à-vis de l'oedème provoqué par Différine® gel, et que seul le sel de potassium de l'acide 18β-glycyrrhétinique est efficace pour diminuer l'irritation due à l'adapalène.

Il est également à noter qu'aucune perte de poids n'est notée pendant l'étude.

### Exemple 3: Evaluation de l'activité comédolytique de Différine® gel seul comparé à Différine® gel associé à un placebo comprenant un anti-irritant chez la souris Rhino

La présente étude a pour but de comparer l'activité comédolytique de Différine® gel (gel de référence à 0,1% d'adapalène) lorsque ce traitement est précédé ou non d'un traitement par un anti-irritant.

Le traitement consiste en une application topique quotidienne d'un véhicule hydro-alcoolique (éthanol/eau 50 :50) comprenant l'anti-irritant sel de potassium de l'acide 18β-glycyrrhétinique, suivie 30 minutes après par une application de Différine® gel, sur la peau du dos de la souris RHINO FVB/N RJ-hr^{rh} (Rhino) pendant 18 jours.

Les produits à tester sont :
Groupe 1 : Différine® gel seul
Groupe 2 : Véhicule hydro-alcoolique puis placebo de Différine® gel
Groupe 3 : Véhicule hydro-alcoolique puis Différine® gel
Groupe 4 : Sel de potassium de l'acide 18β-glycyrrhétinique puis Différine® gel

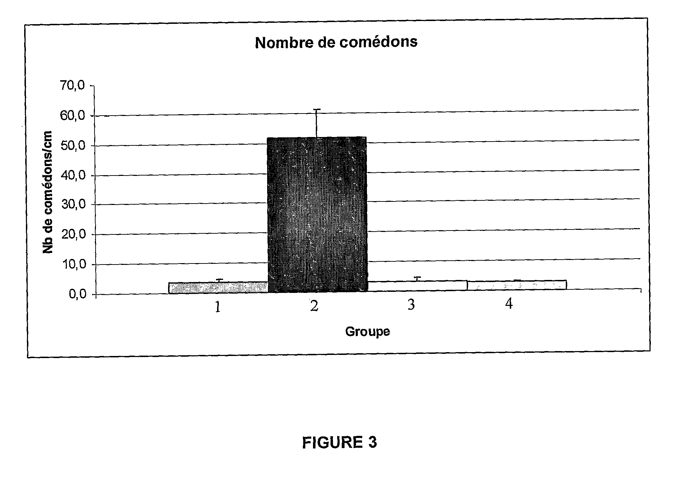

La figure 3 représente les résultats du comptage du nombre de comédons par centimètre (cm) sur le dos des souris Rhino après 18 jours de traitement topique pour les 4 groupes mentionnés précédemment.
Les résultats de cette étude montrent que les peaux traitées avec des placebos (groupe 2) présentent un nombre élevé de comédons par centimètre, compris entre 51 et 60. Les peaux traitées avec Différine® gel seul ou précédé d'un placebo (groupes 1 et 3) présentent un nombre comparable et faible de comédons par centimètre, compris entre 3 et 5.
Les peaux préalablement traitées avec l'anti-irritant sel de potassium de l'acide 18β-glycyrrhétinique présentent un nombre de comédons par centimètre statistiquement équivalent à celui des groupes 1 et 3.

Il ressort donc de la figure 3 que l'anti-irritant sel de potassium de l'acide 18β-glycyrrhétinique avec Différine® gel en traitement dissocié ne diminue pas l'activité comédolytique de Différine® gel seul.

Enfin, après 18 jours de traitement topique, il est à noter que les animaux ne présentent pas de perte de poids.

Cette étude globale démontre que l'application du sel de potassium de l'acide 18β-glycyrrhétinique spécifique avant le traitement au Différine® gel ne diminue pas l'activité comédolytique de Différine® gel.

### Exemple 4 : Formulation de type gel comprenant adapalène à 0,1% et anti-irritants

| **Ingrédients** | **Formule A** |
|---|---|
| Adapalène | 0.1% |
| Eau purifiée | 80.0% |
| Sel de potassium de l'acide 18β-glycyrrhétinique | 1.5% |
| Titriplex III | 0.2% |
| Natrosol 250 HHX Pharm | 2.0% |
| Propylène glycol | 4.0% |
| Synperonic PE/L62 | 0.2% |
| Phénoxyéthanol | 1.0% |
| Eau purifiée | Qsp 100% |

### Exemple 5 : Etude de tolérance de la formulation de l'exemple 4

Une étude de tolérance est menée selon le protocole de l'exemple 2 avec la formulation de l'exemple 4. Cependant, il s'agit ici, contrairement à l'exemple 2, d'un traitement non dissocié, puisque l'adapalène et l'anti-irritant sont présents dans la même formulation.

Les résultats des aires sous la courbe (AUC) des cinétiques d'épaisseur d'oreille entre les jours 2 et 19 sont donnés dans le tableau suivant :

| | **AUC J2-J19** | | **% Augmentation Vs placebo** | **Student t-test Vs placebo** | **% Inhibition Vs Différine gel** |
|---|---|---|---|---|---|
| | **Moyenne** | **Ecart-type** | | | |
| **Différine Placebo** | 358.0 | 3.8 | | | |
| **Différine Gel 0.1%** | 519.5 | 25.8 | 45.1 | *** | |
| **Formule A** | 402.4 | 17.0 | 12.1 | * | 22.5 |

### Conclusions de l'étude :

- Différine*®* Gel 0.1 % augmente l'aire sous la courbe de 45% par rapport au gel placebo.
- La formulation avec l'anti-irritant augmente l'aire sous la courbe par rapport au gel placebo de 12%.
- Par rapport au Différine*®* Gel 0.1 %, la formule A est moins irritante de 20%.
- Le sel de potassium de l'acide 18β-glycyrrhétinique semble être un anti-irritant efficace. Ces résultats confirment ceux de l'exemple 2 où l'anti-irritant avait été évalué en traitement dissocié, c'est-à-dire avant application de Différine® Gel 0.1%.

## Revendications

1. Composition comprenant, dans un milieu physiologiquement acceptable, au moins un composé rétinoïde qui est l'adapalène,
et au moins un composé anti-irritant qui est le sel de potassium de l'acide 18β-glycyrrhétinique.

2. Composition selon la revendication 1 **caractérisée en ce qu'**elle se présente sous la forme d'un gel.

3. Composition selon l'une des revendications 1 à 2, **caractérisée en ce que** la concentration en composé rétinoïde est comprise entre 0,001% et 10%, préférentiellement entre 0,01% et 5% et, plus préférentiellement, entre 0,05% et 2% en poids du poids total de la composition.

4. Composition selon l'une des revendications 1 à 3, **caractérisée en ce que** la concentration en composé rétinoïde est égale à 0,1%.

5. Composition selon l'une des revendications 1 à 4, **caractérisée en ce que** la concentration en composé rétinoïde est égale à 0,3%.

6. Composition selon l'une des revendications 1 à 5, **caractérisée en ce que** la concentration en composé anti-irritant est comprise entre 0,01% et 10%, préférentiellement entre 0,1% et 7%.

7. Composition selon l'une quelconque des revendications précédentes à titre de médicament.

8. Utilisation d'une composition selon l'une des revendications 1 à 6 pour la préparation d'un médicament destiné au traitement et/ou à la prévention des affections dermatologiques liées à un désordre de la kératinisation portant sur la différenciation et sur la prolifération cellulaire notamment pour traiter les acnés vulgaires, comédoniennes, papulopustuleuse, papulocomédoniennes, nodulokystiques, les acnés conglobata, les acnés chéloïdes de la nuque, les acnés miliaires récidivantes, les acnés nécrotiques, les acnés neonatorum, les acnés professionnelles, les acnés rosacées, les acnés séniles, les acnés solaires et les acnés médicamenteuses.

9. Utilisation d'une composition selon la revendication 8 pour la préparation d'une composition pharmaceutique destinée à prévenir ou à traiter les acnés vulgaires.

10. Utilisation cosmétique non thérapeutique d'une composition selon l'une quelconque des revendications 1 à 6 pour lutter contre l'aspect gras de la peau ou des cheveux.

## Patentansprüche

1. Zusammensetzung, umfassend in einem verträglichen physiologischen Milieu wenigstens eine Retinoidverbindung, die Adapalen ist, und wenigstens eine nicht irritierende Verbindung, die das Kaliumsalz der 18β-Glycyrrhetinsäure ist.

2. Zusammensetzung gemäß Anspruch 1, **dadurch gekennzeichnet, dass** sie in Form eines Gels vorliegt.

3. Zusammensetzung gemäß einem der Ansprüche 1 bis 2, **dadurch gekennzeichnet, dass** die Konzentration der Retinoidverbindung zwischen 0,001 Gew.-% und 10 Gew.-%, bevorzugt zwischen 0,01 Gew.-% und 5 Gew.-% und bevorzugter zwischen 0,05 Gew.-% und 2 Gew.-% des Gesamtgewichts der Zusammensetzung beträgt.

4. Zusammensetzung gemäß einem der Ansprüche 1 bis 3, **dadurch gekennzeichnet, dass** die Konzentration der Retinoidverbindung 0,1% beträgt.

5. Zusammensetzung gemäß einem der Ansprüche 1 bis 4, **dadurch gekennzeichnet, dass** die Konzentration der Retinoidverbindung 0,3% beträgt.

6. Zusammensetzung gemäß einem der Ansprüche 1 bis 5, **dadurch gekennzeichnet, dass** die Konzentration der nicht irritierenden Verbindung zwischen 0,01% und 10%, bevorzugt zwischen 0,1% und 7%, beträgt.

7. Zusammensetzung gemäß einem der vorhergehenden Ansprüche als Medikament.

8. Verwendung einer Zusammensetzung gemäß einem der Ansprüche 1 bis 6 zur Herstellung eines Arzneimittels zur Behandlung und/oder Prävention dermatologischer Erkrankungen, die mit einer Störung der Keratinisierung im Zusammenhang mit der Zelldifferenzierung und Zellproliferation verbunden sind, insbesondere zur Behandlung der Acne vulgaris, Acne comedonica, Acne papulopustulosa, Acne papulocomedonica, Acne nodulocystica, Acne conglobata, Acne scleroticans nuchae, rezidivierende Acne miliaris, Acne necrotica, Acne neonatorum, Acne professionalis, Acne rosaceae, Acne senilis, Acne solaris und Acne medicamentosa.

9. Verwendung einer Zusammensetzung gemäß Anspruch 8 zur Herstellung einer pharmazeutischen Zusammensetzung zur Prävention oder Behandlung der Acne vulgaris.

10. Nichttherapeutische kosmetische Verwendung einer Zusammensetzung gemäß einem der Ansprüche 1 bis 6 zur Bekämpfung des fettigen Aussehens von Haut oder Haaren.

## Claims

1. A composition comprising, in a physiologically acceptable medium, at least one retinoid compound which is adapalene, and at least one anti-irritation compound which is the potassium salt of 18β-glycyrrhetinic acid.

2. The composition according to claim 1, **characterized in that** it appears as a gel.

3. The composition according to one of claims 1 to 2, **characterized in that** the retinoid compound concentration is comprised between 0.001% and 10%, preferentially between 0.01% and 5% and, more preferentially, between 0.05% and 2% by weight based on the total weight of the composition.

4. The composition according to one of claims 1 to 3, **characterized in that** the retinoid compound concentration is equal to 0.1%.

5. The composition according to one of claims 1 to 4, **characterized in that** the retinoid compound concentration is equal to 0.3%.

6. The composition according to one of claims 1 to 5, **characterized in that** the anti-irritation compound concentration is comprised between 0.01% and 10%, preferentially between 0.1% and 7%.

7. The composition according to any one of the preceding claims, as a drug.

8. The use of a composition according to one of claims 1 to 6, for preparing a drug intended for treating and/or preventing dermatological diseases related to a keratinization disorder dealing with cell differentiation and proliferation, notably for treating acne vulgaris, comedonal, papulopustular, papulocomedonal, nodulocystic acnes, conglobata acnes, keloidal acne at the nape of the neck, recurrent miliary acne, necrotic acnes, acne neonatorum, professional acnes, rosacea acnes, senile acnes, solar acnes and drug-related acnes.

9. The use of a composition according to claim 8, for preparing a pharmaceutical composition intended for preventing or treating acne vulgaris.

10. The non-therapeutic cosmetic use of a composition according to any one of claims 1 to 6 for combating the fatty aspect of skin or hair.
